# EUROPEAN PATENT APPLICATION

(11) **EP 4 049 621 A1**
(43) Date of publication of application: **31.08.2022**
(21) Application number: 21839310.6
(22) Date of filing: 18.01.2021
(51) Int. Cl.: A61F 2/24

(54) **ARTIFICIAL VALVE PROSTHESIS**

(30) Priority: 28.12.2020 CN 202011606843; 28.12.2020 CN 202023228721 U
(71) Applicant: Shanghai Trulive Medtech Co., Ltd, Shanghai 201206 (CN)
(72) Inventor: YANG, Wei, Shanghai 201206 (CN)
(74) Representative: Argyma
(86) International application number: PCT/CN2021/072485
(87) International publication number: WO 2022/141679

(57) **Abstract**

A prosthetic valve prosthesis including a stent body and a valve leaflet assembly is disclosed. The stent body includes a first stent and a second stent. The first stent is provided with a connecting end. The second stent includes several stent rods, and the several stent rods are fixed to the connecting end of the first stent respectively. The valve leaflet assembly is provided with a first valve leaflet fixing portion, and the valve leaflet assembly is fixed to the connecting end of the first stent by the first valve leaflet fixing portion.

## Description

### BACKGROUND OF THE INVENTION

### Field of the Invention

This invention relates to the technical field of medical instruments, in particular, to a transcatheter prosthetic heart valve prosthesis.

### Description of the Prior Art

The heart contains four cavities, i.e., right atrium (RA), right ventricle (RV), left atrium (LA), and right ventricle (LV). During the whole cardiac cycle, pumping on the left and right sides of the heart usually occurs synchronously. A valve dividing the atrium from the ventricle is called an atrioventricular valve. The atrioventricular valve functions as a one-way valve to ensure normal blood flow in the cardiac cavity. The atrioventricular valve between the left atrium and the left ventricle is a mitral valve, and the atrioventricular valve between the right atrium and the right ventricle is a tricuspid valve. A pulmonary valve directs the blood flow to a pulmonary artery and from there to the lung; the blood returns to the left atrium through the pulmonary vein. An aortic valve directs the blood flow through an aorta and from there to the surrounding regions. Normally, there is no direct connection between the atriums or the ventricles.

When the ventricle begins to inflate (diastole), the aortic valve and the pulmonary valve close to prevent regurgitation from the artery to the ventricle. Shortly thereafter, the atrioventricular valve opens to allow unobstructed flow from the atrium into the corresponding ventricle. Shortly after the ventricular systole (i.e., the ventricular emptying) begins, the tricuspid valve and the mitral valve normally close, thereby forming a seal that prevents the regurgitation from the ventricle to the corresponding atrium.

When there is a problem with the atrioventricular valve, the functions cannot be exerted properly, resulting in improper shutdown. The atrioventricular valve is of a complicated structure, which usually includes the valve annulus, the valve leaflets, the chordae tendineae, and the support structure. Each of the atriums is connected to the valve thereof by an atrium vestibule. The mitral valve has two leaflets while the similar structure of the tricuspid valve has three valve leaflets, and the attachment or engagement between the corresponding surfaces of the valve leaflets facilitates the closure or the seal of the valve, thereby preventing the blood from flowing in an incorrect direction. During the ventricular systole, a situation where the valve leaflets cannot be sealed is called a poor engagement, which causes the blood to flow reversely through the valve (i.e., the regurgitation). The valvular regurgitation may cause severe results for the patients, typically resulting in heart failure, reduction of the blood flow amount, reduction of blood pressure and/or reduction of oxygen flow amount reaching the human tissues. The mitral regurgitation may further cause the blood to flow from the left atrium back to the pulmonary vein, thereby resulting in hyperemia. A severe valvular regurgitation, if not treated, may cause permanent disability or death.

The left ventricular outflow tract obstruction (LVOTO) is mainly caused by narrow flowing tracts when the left ventricle ejects the blood outside due to the thickened interventricular septum and asymmetrical thickening of the interventricular septum for the patients with hypertrophic cardiomyopathy. The blood pass through narrow regions when the heart shrinks. Since the force at the narrow region is tremendous and the native valve leaflets are replaced or repaired to be abutted to the two sides, this force absorbs the native mitral valve to the interventricular septum to make the narrow situation more serious. In the later stages of the heart contraction, the native valve leaflets may further completely obstruct the outflow tract of the blood to form the left ventricular outflow tract obstruction. Common symptoms of left ventricular outflow tract obstruction are being flustered, shortness of breath and lack of strength after activities, even angina and syncope with episodes of angina, and heart failure in the later period of the disease.

The thrombus is a small piece of blood flow formed on the surface of the blood vessels in the cardiovascular system where it peels off or repairs. In the variable fluid-dependent patterns, the thrombus consists of insoluble fibrin, deposited platelets, accumulated white blood cells, and trapped red blood cells. When the endomembrane is damaged, the endothelial cells will degrade, form necrosis and fall off, and the subendothelium collagenous fiber is exposed, so that XII factors of the intrinsic pathway for blood clotting are activated. When the intrinsic pathway for blood clotting is activated, the damaged endomembrane may release coagulation factors of tissues to activate the extrinsic pathway for blood clotting. The damaged endomembrane becomes rough so that the platelets are easy to gather and adhere mainly to the exposed collagen fibers.

In current years, there have been some breakthroughs in the field of the prosthetic valve, but the treatment of the mitral valve still faces great challenges due to the complexity of the mitral valve and the surrounding structures. For example: 1. How to deal with the deposition of the blood flow in the positions where the valve leaflets are connected with the stent and how to avoid the thrombosis in these positions; 2. How to reduce the diameter of the apparatus for delivering the valve to reduce the trauma area when being implanted; and, 3. How to avoid the outflow tract from being obstructed by the native valve leaflets and how to solve the problem of the outflow tract obstruction.

### SUMMARY OF THE INVENTION

This invention provides a prosthetic valve prosthesis, which may solve the above drawbacks in the prior art.

The technical solution of this invention is as follows:
A prosthetic valve prosthesis includes a stent body and a valve leaflet assembly,
wherein the stent body includes a first stent and a second stent that are configured to be provided with a mesh frame structure, the first stent being provided with a connecting end, and wherein the second stent includes several stent rods, the several stent rods are fixed to the connecting end of the first stent and the valve leaflet assembly is configured with a first valve leaflet fixing portion, the first valve leaflet fixing portion being fixed to the connecting end of the first stent.

Compared with the solution in the prior art where the stent body is a frame structure consisting of several enclosed geometric units, the first stent of this invention is a mesh frame structure to ensure that the stent body may be compressed into a delivery apparatus, the second stent of this invention mainly consists of several stent rods, so that the amount of the stent body material of this invention is reduced, thereby reducing a cross section area of a delivery apparatus and reducing a trauma area of a patient; in addition, the stent structure of a region sandwiched between the valve leaflets and the second stent is on a side deviated from the first stent and is relatively sparse, so that the blood is not easily deposited there, thereby reducing a risk of thrombosis. Further, when the second stent is anchored in the ventricle, the sparse stent structures further facilitate the reduction of the risk of obstructing or blocking the outflow tract.

In some embodiments, the first valve leaflet fixing portion is configured to have an extension arc matched with the connecting end, so that the first valve leaflet fixing portion coincides with the connecting end. Then, the region sandwiched between the valve leaflet assembly and the stent, which is deviated from the first stent, are entirely sparse structures formed by the stent rods, which may prevent the blood from depositing in this region.

In some embodiments, the stent rods are fixed on an end portion (one end portion or two end portions) of the connecting end, or the stent rods are disposed by way of being configured between two adjacent valve leaflets. Preferably, when the valve leaflet assembly is configured with a plurality of valve leaflets, a joint between two adjacent valve leaflets is configured to be a second valve leaflet fixing portion and the valve leaflet assembly is further fixed on the stent rod by the second valve leaflet fixing portion. With this structure, the stent rods may provide fixing positions to the valve leaflets and provide corresponding acting forces for opening and closing the valve leaflets; the two adjacent valve leaflets are fixed to the stent rods at the joint between the two adjacent valve leaflets by the second valve leaflet fixing portion, so that the amplitude of opening the valve leaflets and the force of patting tissues when the valve leaflets open are reduced, thereby lengthening the service life of the valve leaflets.

Further, the number of the stent rods is 2, preferably 2 to 5. When the number of the stent rods is too large, the amount of the material for the stent body may be increased and the cross-section area of the delivery apparatus may be increased, thereby increasing the trauma area; when the number of the stent rods is too small, enough fixing forces may not be provided to the valve leaflets to ensure the smooth opening and closure of the valve leaflets.

In some embodiments, the stent rod is configured to be provided with a first end portion fixed with the first stent, the stent rod is further provided with a valve leaflet attachment portion for fixing valve leaflets, and a distance from the valve leaflet attachment portion to an end surface of the first end portion accounts for 1/12 to 1/2 of a length of the stent rod. Since the area of the first stent is too large, the valve leaflets are stitched to the second stent close to the first stent, which facilitates the first stent to share the action force on the stent rods exerted by the valve leaflets and increases the endurance of the stent rods.

Preferably, the first end portion is configured to be in a columnar shape or a trumpet shape; when the first end portion is configured to be in the trumpet shape, the stent rods and the first stent are too smooth, and the force of the stent rods are spread evenly over the entire first stent, thereby increasing the endurance of the stent body. Further, the valve leaflet attachment portion is configured to be several stitching holes, wherein preferably, the stitching holes are of circular holes, and the number of the stitching holes is 1 to 10, preferably 2 to 5.

In some embodiments, a free end of at least one of the stent rods is configured to be a hook portion for hooking native valve leaflets; the hook portion fixes the native valve leaflets to prevent the outflow tract from being obstructed by the native valve leaflets, and is also used to hook the tissues to play a certain role of anchoring. Preferably, at least two of the stent rods in the second stent are configured with the hook portions, wherein the hook portion should be disposed symmetrically, so as to fix the native valve leaflets more effectively and anchor the stent body more stably.

In some embodiments, an included angle γ between the second stent and the first stent ranges from 10 ° to 175 ° , and preferably, the included angle γ ranges from 90° to 160° . With this included angle, the first stent may be combined with the atrium closely, and the atrium may provide enough anchoring sites to the stent.

In some embodiments, the second stent further includes a valve leaflet cutting member for cutting the native valve leaflets; the valve leaflet cutting member cuts the native mitral valve to be hooked by the stent rods on two sides, so as to prevent the outflow tract from being obstructed by the native valve leaflets.

Preferably, the valve leaflet cutting member is fixed to the connecting end of the first stent, and is located between two adjacent stent rods, so that the native valve leaflets may be fixed by the hook portion of the stent rods on two sides after being cut.

In some embodiments, the valve leaflet cutting member is configured to extend towards a direction deviated from the valve leaflet assembly, and an included angle α between the valve leaflet cutting member and an extension direction of the stent rod ranges from 0° to 90° , and further preferably the included angle α ranges from 0° to 45 ° . With this design, it is ensured that the valve leaflet cutting member have cut the native valve leaflets before the stent rods are hooked to the native valve leaflets, so that the outflow tract may be avoided from being obstructed by the oversized native valve leaflets while this included angle may ensure that the valve leaflet cutting member does not interfere with the normal opening and closing of the replacement valve leaflets.

In some embodiments, the valve leaflet cutting member is configured with several cutting portions, and each of the cutting portions is in a triangle shape or a square shape or provided with an arc-shaped cutting edge.

The cutting portions are distributed from the free end of the valve leaflet cutting member to the other end portion of the valve leaflet cutting member; preferably, along a direction towards which the valve leaflet cutting member extends, the several cutting portions are distributed in a continuous manner or the several cutting portions are distributed in a discontinuous manner.

In some embodiments, the prosthetic valve prosthesis is used to replace a native leaflet with lesions, such as the anterior leaflet or the posterior leaflet of the mitral valve, or to repair the tricuspid valve and the aortic valve, and then the stent body is configured to be of a non-enclosed structure; when the valve prosthesis is used to replace the native valve, the stent body is configured to be of an annular structure enclosed circumferentially.

Compared with the existing technology, the present invention has the following beneficial effects:
First, according to the prosthetic valve prosthesis of this invention, the second stent mainly consists of several stent rods, and the valve leaflet assembly is fixed to the second stent; compared with the solution where the stent body is a frame structure consisting of several enclosed geometric units in the prior art, the provision of the stent rods in this invention reduces the amount of the stent body material, thereby reducing a cross section area of a delivery apparatus and reducing a trauma area of a patient; in addition, in the region sandwiched between the valve leaflets and the second stent, which is deviated from the first stent, the blood is not easy to be deposited here, thereby reducing the risk of thrombosis; moreover, when the second stent is anchored in the ventricle, the structure of the stent rod facilitates the reduction of obstacle or blockade of the outflow tract.

Second, according to the prosthetic valve prosthesis of this invention, the connecting end of the first stent is configured to have the arc matched with the valve leaflet fixing end, and the first valve leaflet fixing portion completely coincides with the connecting end of the first stent after the valve leaflet assembly is fixed; at a side deviated from the first stent, the region sandwiched between the valve leaflet assembly and the second stent is of a relatively-sparse structure consisting of the stent rods, so that the blood will not be deposited here, thereby further reducing the risk of thrombosis; at the same time, the amount of the stent body material is further reduced, and the cross section area of the delivery apparatus is reduced.

Third, according to the prosthetic valve prosthesis of this invention, the free ends of the stent rods are further configured with a hook portion for hooking the native valve leaflets to form a retention force for the native valve leaflets, so that the outflow tract is prevented from being blocked by the native valve leaflets when the heart contracts, and the hook portion may also be used for hooking the tissues to play a certain anchoring role; the second stent is further configured with the valve leaflet cutting member, which cut the native valve leaflets and may be hooked by the stent rods on two sides, so that the outflow tract is avoided from being blocked by the larger native valve leaflets.

Certainly, any one product for implementing the present invention is unnecessary to achieve all the above advantages at the same time.

### BRIEF DESCRIPTION OF THE DRAWINGS

Fig. 1 is an overall structural diagram of the valve prosthesis according to Embodiment 1 of this invention, wherein Fig. 1A is a structural diagram of a stent body, and Fig. 1B is a structural diagram of a valve leaflet assembly;
Fig. 2 is an overall structural diagram of the stent body in the prior art;
Fig. 3 is an overall structural diagram of a stent rod according to Embodiment 1 of this invention;
Fig. 4 is a local structural diagram of the stent body according to Embodiment 1 of this invention;
Fig. 5 is a local structural diagram of another stent body according to Embodiment 1 of this invention;
Figs. 6A-6D are structural diagrams of a valve leaflet cutting member according to Embodiment 1 of this invention, respectively.

Reference for numerals: first stent 110; valve leaflet assembly 130; second stent 120; stent rod 121; first valve leaflet fixing portion 134; second valve leaflet fixing portion 135; valve leaflets (131, 132, 133); connecting end 210; first connecting end 211; second connecting end 212; third connecting end 213; first end portion 120-1; valve leaflet attachment portion 120-2; hook portion 120-3; valve leaflet cutting member 140; cutting portion 141.

### DESCRIPTION OF THE PREFERRED EMBODIMENTS

In the description of the present invention, it should be noted that orientations or position relationships indicated by terms "center", "upper", "lower", "left", "right", "vertical", "horizontal", "inside", "outside" and the like are orientations or position relationships shown in the drawings, and these terms are merely for facilitating description of the present invention and simplifying the description, but not for indicating or implying that the mentioned apparatus or elements must have a specific orientation and must be established and operated in a specific orientation, and thus, these terms cannot be understood as a limitation to the present invention. Moreover, terms like "first", "second", "third" etc. are only used for description, not be considered as a designation or designation of relative importance.

In the description of the present invention, it should be noted that, unless otherwise clearly specified and limited, meanings of terms "install", "connected with", and "connected to" should be understood in a board sense. For example, the connection may be a fixed connection, a removable connection, or an integral connection; may be a mechanical connection or an electrical connection; may be a direct connection or an indirect connection by using an intermediate medium; or may be intercommunication between two components. For those of ordinary skill in the art, specific meanings of the above terms in the present invention may be understood based on specific situations.

As used in the specification, singular forms "a/an," "one," and "the/that" include plural objects, unless otherwise explicitly stated. As used in the specification, the term "or" is usually used to include the meaning of "and/or", unless otherwise expressly stated.

The following further describes the present invention in combination with specific embodiments.

### Embodiment 1

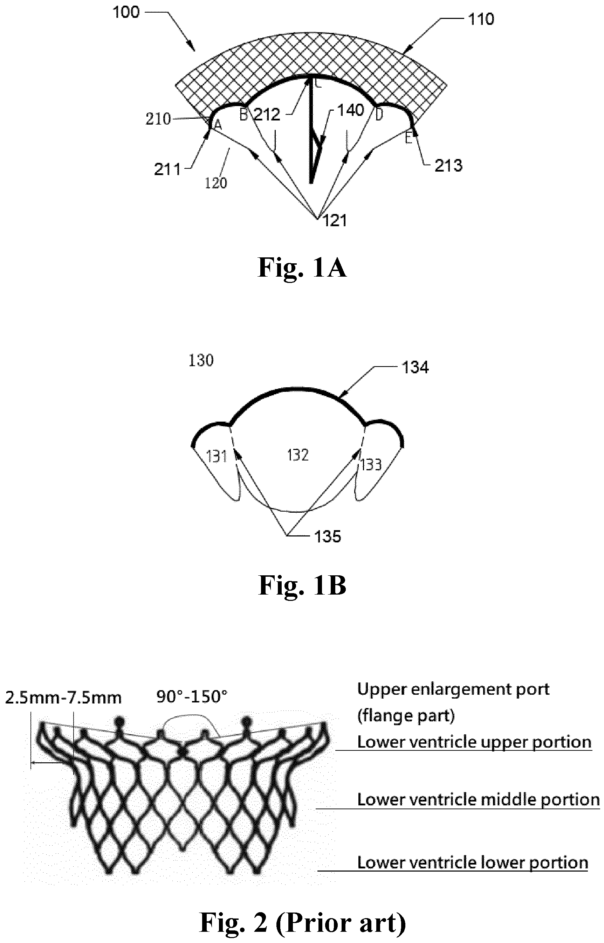

This embodiment provides a prosthetic valve prosthesis (also referred to as a valve prosthesis). With reference to Figs. 1A to 6D, the valve prosthesis including a stent body and a valve leaflet assembly 130. The stent body may be served as a support structure of the valve leaflet assembly 130 while playing an anchoring role for anchoring with the tissues and a connecting role for connecting with the delivery apparatus (e.g., one end portion or two end portions of the stent body provided with a hanging tab or a fixing tab) and so on. The prosthetic valve prosthesis of this embodiment is a valve prosthesis delivered via a catheter, wherein the valve prosthesis is compressed into a delivery apparatus for delivery when being implanted and then released and anchored after being delivered to a target position. The stent body is made from biocompatible materials such as nickel-titanium or cobalt-chromium, and the valve leaflet assembly 130 includes at least one prosthetic valve leaflet. According to actual clinical needs, the corresponding number of prosthetic valve leaflets may be disposed, and the prosthetic valve leaflets may be biological tissues such as bovine pericardia, porcine pericardia, and equine pericardia.

With reference to Fig. 1A, the stent body includes a first stent 110 and a second stent 120, and the first stent 110 is configured to be provided with a mesh frame structure. The first stent 110 is provided with a connecting end 210 and connected with the second stent 120 by the connecting end 210. The second stent 120 includes several stent rods 121, the stent rods 121 are fixed to the connecting end 210 of the first stent 110 respectively, and the valve leaflet assembly 130 is configured with a first valve leaflet fixing portion 134 that is fixed to the connecting end 210 of the first stent 110.

The first stent 110 is configured to be the mesh frame structure formed by arranging several enclosed geometrical units. The enclosed geometrical unit includes, but is not limited to, a triangle shape, a square shape, a pentagon shape, a droplet shape, a heart shape, a diamond shape and so on. Preferably, the mesh frame structure is formed by arranging the diamond shape units, so that the first stent 110 may be compressed into a delivery catheter for delivery, and the first stent 110 self-expands to recover to the original shape after being released. The stent rods 121 may be fixed at apexes of edges of the diamond units of the first stent 110, so that the overall stent body may be compressed while self-expanding.

In the prior art, the stent body is a frame structure formed by arranging several enclosed geometrical units as a whole. With reference to Fig. 2, a local structural diagram of the stent body of the prior art is shown, including an upper enlargement port, a lower ventricle upper portion, a lower ventricle middle portion, and a lower ventricle lower portion, wherein the upper enlargement port is anchored into the atrium, the valve leaflets are fixed to the lower ventricle middle portion of the stent body, and the lower ventricle lower portion is located in the ventricle. The blood flows in from the upper enlargement port while flowing out from the lower ventricle lower portion. After the valve leaflets are fixed, a region is sandwiched at a joint between the frame structure and the valve leaflets and a place close to a side of the lower ventricle lower portion. The region is easy to cause the blood to be deposited to form thrombus.

In order to solve the problem of thrombosis, in the prior art, an anti-thrombus process is typically performed for the materials contacting the blood to reduce the thrombosis, but there is a risk of thrombosis. At the same time, reducing the amount of the material of the valve prosthesis for reducing the diameter of the delivery apparatus is also a major problem encountered with.

Different from the structure of the stent body and the technical concept under which the thrombosis is solved in the prior art, the stent body of this embodiment includes a first stent 110 and a second stent 120, wherein the second stent 120 includes several stent rods 121 fixed to the connecting end 210 of the first stent, and the rod-shaped structure of the stent rod 121 causes the second stent 120 to form into a relatively-sparse stent structure. Different from the solution in the prior art where both the lower ventricle middle portion and the lower ventricle lower portion are of the mesh frame structures, the second stent 120 of this embodiment may not form staggered meshes in an extension direction perpendicular to the stent rod. At the joint between the valve leaflet assembly 130 and the stent body, which is deviated from the first stent 110, the relatively-sparse structure of the second stent 120 causes the blood to be not easily deposited in this region, thereby reducing the risk of thrombosis in this region. Meanwhile, the structure of the second stent 120 further reduces the amount of the stent body material, thereby reducing the cross-section area of delivering the valve prosthesis and reducing the trauma area of the patient.

Further, when the first stent 110 is mainly anchored in the atrium and the second stent 120 is anchored in the ventricle, the structure of the second stent 120 causes the valve prosthesis to be more sparse at the side close to the outflow tract as compared to the stent body in the prior art. Therefore, when the valve prosthesis of this embodiment is used as the mitral valve, the left ventricular outflow tract obstacle may be reduced, and when the valve prosthesis is used as the aortic valve, the left ventricular outflow tract blockade may be reduced.

Since the first valve leaflet fixing portion 134 in the valve leaflet assembly 130 used for fixation has a certain arc, in some embodiments, the connecting end 210 is configured to have an extension arc matched with the first valve leaflet fixing portion 134, so that the first valve leaflet fixing portion 134 coincides completely with the connecting end 210. With this structure, the joint between the valve leaflet assembly 130 and the stent body, which is deviated from the first stent 110 are sparse structures formed by the several stent rods, thereby further reducing the risk of thrombosis in this region.

In this embodiment, the number of the stent rods 121 is 2, preferably 2 to 5. When the number of the stent rods is too large, the amount of the stent body material and the cross-section area of the delivery apparatus are increased, thereby increasing the trauma area; when the number of the stent rods is too small, enough fixing forces may not be provided to the valve leaflets to ensure the smooth opening and closure of the valve leaflets.

The stent rod 121 may be located at any position of the connecting end 210 of the first stent. In some embodiments, the valve prosthesis is used to replace the native valve leaflets with local lesions; the stent body is configured to be of a non-enclosed structure such as a fan structure, which may be used with the cooperation of the native posterior valve leaflets when the mitral valve anterior valve leaflets are repaired and may be used with the cooperation of the native anterior valve leaflets when the mitral valve posterior valve leaflets are repaired. Naturally, this structure may be used to repair the aortic valve or the tricuspid valve, and the stent body and the valve leaflet assembly 130 are configured according to different objects to be repaired.

With continuous reference to Fig. 1A, the connecting end 210 of the first stent has two end portions (A and E) along the extension direction thereof, and the second stent 120 is configured with at least two stent rods 121, wherein the stent rods 121 are preferably fixed to the end portions of the connecting end 210, i.e., may be fixed to one of the end portions of the connecting end 210 or fixed to the two end portions of the connecting end 210. Compared to being fixed to other positions, fixing the stent rods 121 to the end portions may form relatively-stable support for the valve leaflet assembly 130. In some embodiments, when the valve leaflet assembly 130 is configured with a plurality of valve leaflets, the stent rods 121 may further be configured between the two adjacent prosthetic valve leaflets for fixing the two adjacent valve leaflets. The joint between the two adjacent valve leaflets is configured to be a second valve leaflet fixing portion 135, and the valve leaflet assembly 130 is further fixed to the above stent rods 121 by the second valve leaflet fixing portion 135.

With reference to Fig. 1B, the valve leaflet assembly 130 has three prosthetic valve leaflets, i.e., a valve leaflet 131, a valve leaflet 132, and a valve leaflet 133. Edges of the valve leaflet assembly 130 are configured to be the above first valve leaflet fixing portion 134, and the second valve leaflet fixing portions 135 are configured between the valve leaflet 131 and the valve leaflet 132 and between the valve leaflet 132 and the valve leaflet 133 respectively. The connecting end 210 of the first stent 110 has a first connecting end 211, a second connecting end 212 and a third connecting end 213, and the first connecting end 211 and the third connecting end 213 are distributed at two sides of the second connecting end 212 symmetrically, wherein each of the connecting ends is used to be fixedly connected with one of the valve leaflets, and each of the connecting ends is configured to have an extension arc matched with fixing ends of the valve leaflets fixed therewith. The endpoint A of the first connecting end 211 and the endpoint E of the third connecting end 213 are fixed with one of the stent rods 121 respectively, and corresponding positions between the first connecting end 211 and the second connecting end 212 and between the second connecting end 212 and the third connecting end 213 are configured with one of the stent rods 121 respectively, so that the stent rods 121 are configured between two endpoints of the connecting end 210 and between the two adjacent two valve leaflets respectively. After the valve leaflet assembly 130 is fixed, the second valve leaflet fixing portion 135 is fixed to one of the stent rods 121 respectively. The stent rods 121 may provide fixing positions for the valve leaflets to provide corresponding acting forces for opening and closing the valve leaflets, so that the amplitude of opening the valve leaflets is reduced and the acting force beating the tissues while the valve leaflets opening is reduced, thereby increasing the service life of the valve leaflets. In addition, the stent rods 121 may provide stable support for the valve leaflet assembly 130, avoiding the valve leaflets from removing from the stent rods when being closed.

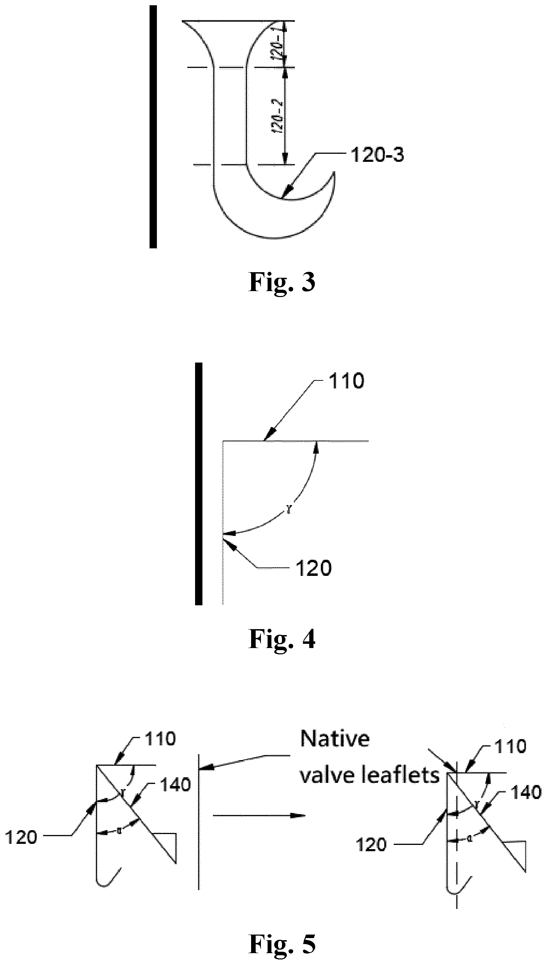

The second stent 120 may provide fixing positions for the valve leaflets, and optionally the fixing positions may be any position located on the stent rods 121. Specifically, with reference to Fig. 3, in some embodiments, the stent rod 121 is configured to be provided with a first end portion 120-1 fixed with the first stent 110, the stent rod 121 is further provided with a valve leaflet attachment portion 120-2 for fixing valve leaflets and a distance from the valve leaflet attachment portion 120-2 to an end surface of the first end portion 120-1 accounts for 1/12 to 1/2 of a length of the stent rod 121. Due to the larger area of the first stent 110, the valve leaflet assembly 130 is stitched to a middle-upper position of the stent rod 121, which facilitates the first stent 110 to share the acting force of the stent rod 121 exerted by the valve leaflet assembly 130.

Further, the valve leaflet attachment portion 120-2 is configured to have several stitching holes, and the stitching holes are in a diamond shape, a circle shape, a triangle shape, and so on, preferably the circle shape. Circle-shaped holes may disperse the force of the stent rod exerted by the valve leaflet assembly 130 on the stent rod 121 uniformly, thereby increasing the endurance property of the stent rod 121. The number of the stitching holes may be 1 to 10, preferably 2 to 5. The more the number of the stitching holes is, the weaker the strength of the stent rod is; the less the number of the stitching holes is, the weaker the supporting force of the valve leaflet assembly 130 exerted by the stent rod is.

Further, the first end portion 120-1 may be configured to be in a columnar shape or a prism shape (e.g., quadrangular prism), and preferably the first end portion 120-1 may be configured to be in a trumpet shape. As shown in Fig. 3, the trumpet shape may cause the stent rods 121 and the first stent 110 to be too smooth, so that the force applied on the second stent 120 may be dispersed uniformly over the entire first stent, thereby increasing the endurance of the stent body.

In some embodiments, the shape of the free end of the stent rod 121 may be a cylindrical shape or a conical shape; preferably, the free end of the stent rod 121 is configured to be the hook portion 120-3 of a hook shape. The hook portion 120-3 of the free end of the stent rod 121 may hook the native valve leaflets or the tissues to play a fixing role for the stent body, and the movement range of the native valve leaflets is limited, thereby further reducing the risk of blocking the outflow tract by the native valve leaflets.

In some preferred embodiments, at least two of the stent rods 121 in the second stent 120 are configured with the hook portion 120-3. The more the number of the hook portion 120-3 is, the stronger the force applied to the native valve leaflets is, preventing the outflow tract obstacle during the cardiac systole due to the native valve leaflets. Preferably, the stent rods 121 configured with the hook portion 120-3 should be configured at two sides of a middle point C of the connecting end 210 of the first stent symmetrically. As shown in Fig. 1, when the connecting rod 120 at the connecting end A of the first stent 110 is configured with the hook portion, the connecting rod 120 at the E is also configured with the hook portion correspondingly. The hook portions are distributed symmetrically, so that the force applied to the stent body by the native valve leaflets or the tissues is easier to be balanced to anchor the stent more stably.

In some embodiments, an included angle γ between the second stent 120 and the first stent 110 ranges from 10° to 175 ° , and preferably, the included angle γ ranges from 90° to 160° , as shown in Fig. 4. With this included angle, the first stent 110 may be combined with the atrium closely, and the atrium may provide enough anchoring sites to the first stent 110.

In a preferred embodiment, the second stent 120 further includes a valve leaflet cutting member 140 for cutting the native valve leaflets; for example, when the mitral valve is repaired, the larger native valve leaflets may be cut by the valve leaflet cutting member 140 to avoid the outflow tract from being blocked by the oversized native valve leaflets, with reference to Figs. 1A and 5. Further, the valve leaflet cutting member 140 is fixed to the connecting end 210 of the first stent 110, and fixed between the two adjacent stent rods 121; the stent rods 121 located at two sides of the valve leaflet cutting member 140 should be configured with the above hook portion, so that the stent rods 121 at the two sides may hook the native valve leaflets after the valve leaflet cutting member 140 cuts the native valve leaflets.

The side where the valve leaflet assembly 130 locates is served as an inner side of the stent body or the valve prosthesis. In some embodiments, the valve leaflet cutting member 140 is configured to extend towards a direction deviated from the valve leaflet assembly 130, and with reference to Fig. 4, the valve leaflet cutting member 140 extends towards an outer side of the stent body; and an included angle α between the valve leaflet cutting member 140 and an extension direction of the stent rod 121 ranges from 0° to 90° , and further preferably the included angle α ranges from 0° to 45° . With this design, it is ensured that the valve leaflet cutting member 140 has cut the native valve leaflets before the stent rods 121 hooks the native valve leaflets, so that the native valve leaflets are effectively fixed for preventing the outflow tract from being blocked by the oversized valve leaflets. Meanwhile, with this angle, the valve leaflet cutting member 140 is far away from the valve leaflet assembly 130 to ensure that the valve leaflet cutting member 140 may not interfere with the normal opening and closure of the replacement valve leaflets.

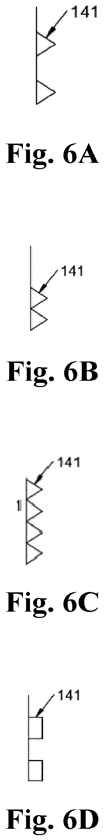

In some embodiments, the valve leaflet cutting member 140 is configured with several cutting portions 141. With reference to Figs. 6A to 6D, the cutting portions 141 may be configured to be in a triangle shape or a square shape or provided with an arc-shaped cutting edge; the specific form of the cutting portions 141 may be configured according to the actual clinical needs. Specifically, to prevent the delivery apparatus or the tissues from being damaged by the cutting portions 141, the cutting portions 141 may be configured to be of a smooth structure. Further, to achieve better cutting effects, the cutting portions 141 may be configured to be provided with a sharp cutting structure, e.g., with a sharp cutting tip or with blade-shaped cutting parts, and then the cutting portions 141 may also be selectively made from biodegradable materials, e.g., polylactic acid, which may be degraded after being cut.

In some embodiments, if the cutting portions 141 are disposed in a continuous manner along the direction in which the valve leaflet cutting member 140 extends, as shown in Figs. 6B and 6C, the cutting portions 141 are adjoined in sequence, and the continuously-disposed cutting portions may make the cutting more smooth. In some embodiments, if several cutting portions 141 are disposed in a non-continuous manner along the direction in which the valve leaflet cutting member 140 extends, as shown in Figs. 6A and 6D, the cutting portions 141 are disposed at intervals, and the non-continuously-disposed cutting portions may provide larger cutting force.

With continuous reference to Fig. 1, the valve leaflet cutting member 140 is fixed at 1/12 to 11/12 of the second connecting end 212, and preferably the valve leaflet cutting member 140 is fixed to a middle point C of the second connecting end 212. This position may disperse the force applied to the first stent 110 by the valve leaflet cutting member 140 uniformly on the left and right sides of the first stent 110.

In a specific embodiment, the cutting portions 141 may be configured to be received in the valve leaflet cutting member 140 when in an implanted state, and when in use, the cutting portions 141 may be exposed by an operation of rotating a handle for achieving the cutting operation.

In some embodiments, the prosthetic valve is used to replace the mitral valve, the tricuspid valve, or the aortic valve, and then the stent body should be constructed as an annular enclosed structure. In other words, the first stent 110 is constructed as the annular enclosed structure circumferentially, the valve leaflet assembly 130 is fixed to an inner circumference side of the annular stent body, and the stent rods 121 of the second stent 120 are distributed circumferentially along the first stent 110. At this time, the stent body material may be a balloon-expandable material.

Specifically, the first stent 110 and the second stent 120 mentioned above are connected fixedly with each other by way of including, but not limited to, welding, fixing with clips, stitching, or preparing integrally. These two parts are preferably prepared integrally, reducing the preparation processes, enhancing the connection strength, and providing a support force to the activities of the valve leaflets. The valve leaflet assembly 130 is fixedly connected with the second stent 120 by way of including, but not limited to, welding, fixing with clips, or preparing integrally, preferably stitching. Further, to increase the biocompatibility of the second stent 120, biocompatible materials such as PET and PTFE may be covered outside.

According to the valve prosthesis in this embodiment, since the design of the stent rods of the second stent 120 substitutes the mesh frame structure of the lower ventricle middle portion and the lower ventricle lower portion in the prior art, the amount of the stent body material is reduced, and the side of the connection between the valve leaflet assembly and the second stent is the sparse structure formed by the stent rods, so that the blood is not easy to be deposited here, thereby reducing the risk of thrombosis. At the same time, the reduction of the blockade or obstacle of the outflow tract is facilitated.

Compared with a frame structure consisting of several enclosed geometric units in the prior art, the second stent of this invention mainly consists of several stent rods, so that the amount of the stent body material of this invention is reduced, thereby reducing a cross section area of a delivery apparatus and reducing a trauma area of a patient; in addition, the stent structure of a region sandwiched between the valve leaflets and the second stent is relatively sparse, so that the blood is not easily deposited there, thereby reducing a risk of thrombosis.

The above disclosure is only the preferred embodiment of this invention, the preferred embodiment does not describe all the details in detail. It should be understood that these embodiments are only used for illustrating this invention, but not for the limitation of the scope of this invention. This invention is limited only by the claims and its full scope and equivalents.

The present invention selects and specifically describes the embodiments with the purpose of better explain the principle and practical use of the present invention, such that a person skilled in the art can well utilize the present invention. Obviously, according to the contents of this specification, there are many modifications and changes that can be made. In practical application, the improvements and adjustments made by those skilled in the art according to this invention still fall within the scope of protection of this invention. The technical characteristics in the different embodiments above can be combined arbitrarily without conflict.

## Claims

1. A prosthetic valve prosthesis, comprising a stent body and a valve leaflet assembly,
wherein the stent body including a first stent and a second stent that are configured to be provided with a mesh frame structure, the first stent being provided with a connecting end, wherein
the second stent includes several stent rods, the several stent rods are fixed to the connecting end of the first stent, and the valve leaflet assembly is configured with a first valve leaflet fixing portion, the first valve leaflet fixing portion being fixed to the connecting end of the first stent.

2. The prosthetic valve prosthesis according to claim 1, wherein the connecting end of the first stent is configured to have an arc matched with the first valve leaflet fixing portion, so that the first valve leaflet fixing portion coincides with the connecting end.

3. The prosthetic valve prosthesis according to claim 1, wherein the stent rods are configured on an end portion of the connecting end, or the stent rods are disposed by way of being configured between two adjacent valve leaflets.

4. The prosthetic valve prosthesis according to claim 3, wherein when the valve leaflet assembly is configured with a plurality of valve leaflets, a joint between two adjacent valve leaflets is configured to be a second valve leaflet fixing portion, and the valve leaflet assembly is further fixed on the stent rod by the second valve leaflet fixing portion.

5. The prosthetic valve prosthesis according to claim 1, wherein the stent rod is configured to be provided with a first end portion fixed with the first stent, the stent rod is further provided with a valve leaflet attachment portion for fixing valve leaflets and a distance from the valve leaflet attachment portion to an end surface of the first end portion accounts for 1/12 to 1/2 of a length of the stent rod.

6. The prosthetic valve prosthesis according to claim 1, wherein a free end of at least one of the stent rods is configured to be a hook portion for hooking native valve leaflets or tissues; and preferably, at least two of the stent rods in the second stent are configured with the hook portions.

7. The prosthetic valve prosthesis according to claim 1, wherein the second stent further comprises a valve leaflet cutting member for cutting the native valve leaflets.

8. The prosthetic valve prosthesis according to claim 7, wherein the valve leaflet cutting member is fixed to the connecting end of the first stent, and is located between two adjacent stent rods.

9. The prosthetic valve prosthesis according to claim 7, wherein the valve leaflet cutting member is configured to extend towards a direction deviated from the valve leaflet assembly, and an included angle α between the valve leaflet cutting member and an extension direction of the stent rod ranges from 0° to 90° , and preferably the included angle α ranges from 0° to 45° .

10. The prosthetic valve prosthesis according to claim 7, wherein the valve leaflet cutting member is configured with several cutting portions, and each of the cutting portions is configured to be in a triangle shape and a square shape or each of the cutting portions is provided with an arc-shaped cutting edge.

11. The prosthetic valve prosthesis according to claim 10, wherein along a direction towards which the valve leaflet cutting member extends, the several cutting portions are distributed in a continuous manner or the several cutting portions are distributed in a discontinuous manner.

12. The prosthetic valve prosthesis according to claim 1, wherein the stent body is configured to be of a non-enclosed structure, or the stent body is configured to be of an annular structure enclosed circumferentially.
